(19)

Europäisches
Patentamt

European
Patent Office

Office européen
des brevets

(11) **EP 3 892 367 A1**

(12) **EUROPEAN PATENT APPLICATION**

(43) Date of publication:
**13.10.2021   Bulletin 2021/41**

(51) Int Cl.:
***B01J 8/06*** (2006.01)

(21) Application number: **20169126.8**

(22) Date of filing: **09.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Röhm GmbH**
**64295 Darmstadt (DE)**

(72) Inventors:
• **KRILL, Steffen**
**64367 Mühltal (DE)**
• **BALDUF, Torsten**
**64319 Pfungstadt (DE)**
• **RACZEK, Melanie**
**64289 Darmsatdt (DE)**

(74) Representative: **Röhm Patent Association**
**IP Management**
**Deutsche-Telekom-Allee 9**
**64295 Darmstadt (DE)**

(54) **A TUBE BUNDLE REACTOR AND METHOD FOR THE  PRODUCTION OF METHACRYLIC ACID THROUGH THE PARTIAL OXIDATION OF METHACROLEIN**

(57)     In general, the present invention relates to the cooling of a product stream produced by the partial oxidation of a $C_3$ - $C_6$ aldehyde in the tubes of a tube bundle reactor. In particular, the present invention relates to a device for cooling the product stream in the end portions of the tubes by making use of particulated solid material that is located in the end portions. The present invention further relates to a method for using the particulated solid material to cool the product stream at a rate that is sufficient to ensure that autoxidation is supressed when the feed stream exits the further longitudinal portion.

## Description

### Field of the invention

In general, the present invention relates to the production of methacrylic acid through the partial oxidation of methacrolein in a tube bundle reactor. In particular, the present invention relates to a device and method for cooling the methacrylic acid and remaining methacrolein after the partial oxidation step has been carried out in order to suppress autoxidation and postcombustion, or the like, in particular other undesired side reactions. The present invention further relates to the products, notably methacrylic acid and the downstream product methyl methacrylate, that are obtained by the aforementioned device and method, the polymerisation of methyl methacrylate and the use of these products in producing a polymer, notably polymethyl methacrylate.

### Background

Methyl methacrylate is a widely used chemical product, with several million tonnes produced worldwide every year. Various production processes are known for producing methyl methacrylate, as summarised in Krill, Offermanns, and Rühling (2019), Chem. Unserer Zeit, 53. One example of these processes is the so-called $C_4$ process wherein isobutylene or tertiary butylalcohol is partially oxidised in the presence of a suitable catalyst to obtain methacrolein, which in turn is then partially oxidised in the presence of another suitable catalyst to obtain methacrylic acid. In a further step, the methacrylic acid can be esterified with methanol to obtain methyl methacrylate.

It is well known in the art that partial oxidation processes can be performed using a tube bundle reactor. An often-challenging aspect of operating a tube bundle reactor is regulating the temperatures of the feed stream prior to, and the product stream after the partial oxidation step. Some inventions have been published dealing with this challenging objective, including the following:

The object of the invention disclosed in DE 30 42 468 A1 is the cooling of acrolein obtained by partial oxidation in a tube bundle reactor. This is achieved by dividing the tubes of the tube bundle reactor into an additional longitudinal portion and a further longitudinal portion, with the latter downstream to the former. The additional longitudinal portion is filled with a solid catalyst, while the further longitudinal portion is filled with a particulated solid material that serves the purpose of intensifying cooling the acrolein produced in the additional longitudinal portion. An important aspect of the disclosure is that the temperatures of the additional longitudinal portion and the further longitudinal portion are independently regulated by secondary cooling with a cooling medium, preferably a molten salt mixture or highly heat resistant oil.

A related teaching is provided by DE 100 47 693 A1, wherein the extension of the lifetime of a catalyst in a tube bundle reactor is addressed. The aforementioned document teaches to place a particulated solid material in the tubes, as well as in an inlet volume and an outlet volume. The inlet volume and the outlet volume are immediately outside and adjacent to the ends of the tubes. DE 100 47 693 A1, however, does not address the reduction of the negative impact and harm on the reactor itself.

Another approach to regulate the temperature is disclosed in DE 198 06 810 A1, with this application focusing on regulating the temperature of the feed stream prior to it flowing into the tubes. This is achieved by insulating the ends of the tubes at the gas-inflowing side from the gas itself, as the aforementioned ends are generally a very hot part of the reactor which contributes to the heat stream.

Even with the implementation of the above-mentioned disclosures, regulating the temperature of the feed and product streams in a tube bundle reactor remains challenging. This is not only important for productivity, but also for safely operating a tube bundle reactor.

### Objects

An object of the present invention is to at least partially overcome at least one of the disadvantages encountered in the state of the art.

It is a further object of the invention to provide a device for cooling a partially oxidised product stream.

It is a further object of the invention to provide a device, used for partially oxidising a product stream, that requires less downtime for maintenance.

It is a further object of the invention to provide a device, used for partially oxidising a product stream, that has improved safety.

It is a further object of the invention to provide a device, used for partially oxidising a product stream, that has increased productivity.

It is a further object of the invention to provide a device, used for partially oxidising a product stream, that can be used safely at higher temperature.

It is a further object of the invention to provide a device, used for partially oxidising a product stream, which can be used more safely and with increased productivity, even when a used or already somewhat deactivated catalyst is employed. It is a further objective of the invention to provide a method for cooling a partially oxidised product stream when the product stream is no longer in contact with a solid catalyst.

It is a further objective of the invention to provide a method for cooling a partially oxidised product stream in order to suppress autoxidation.

It is a further objective of the invention to provide a method for cooling a partially oxidised product stream to a temperature that is below the autoxidation temperature of the partially oxidised product stream.

## Preferred embodiments of the invention

A contribution to at least partially fulfilling at least one of the above-mentioned objects is made by the independent embodiments. The dependent embodiments provide preferred embodiments which contribute to at least partially fulfilling at least one of the objects.

|1| A reactor assembly arranged in fluid communication comprising a first reactor for the partial oxidation of a $C_3$ - $C_6$ aldehyde, preferably acrolein, more preferably methacrolein, the first reactor comprising as reactor components

> a) a first inlet;
> b) downstream the first inlet an inlet volume;
> c) downstream the inlet volume a first metal plate;
> d) downstream the first metal plate a further metal plate;
> e) downstream the further metal plate an outlet volume;
> f) downstream the outlet volume a first outlet;
> g) a plurality of tubes with a tube length, wherein each of the tubes

>> (i) is located between the inlet volume and the outlet volume,
>> (ii) comprises an inlet-end,
>> (iii) comprises an outlet-end,
>> (iv) comprises an outer surface,
>> (v) comprises an inner surface,
>> (vi) comprises an inner volume confined by the inner surface of the tube;

> and wherein at least 50 %, preferably at least 80 %, and more preferably at least 90 % of the tubes each

>> [I] comprise a first longitudinal portion, wherein the first longitudinal portion is positioned in the first metal plate,
>> [II] comprise an additional longitudinal portion, wherein the additional longitudinal portion

>>> (a) is arranged downstream the first longitudinal portion, and
>>> (b) comprises a first solid catalyst,

>> [III] comprise a further longitudinal portion, wherein the further longitudinal portion

>>> A) is arranged downstream the additional longitudinal portion,
>>> B) is positioned in the further metal plate,
>>> C) comprises an inert particulated solid material, wherein the particulated solid material

>>>> (I) is adapted and arranged to dissipate heat,
>>>> (II) is gas-permeably adapted and arranged;

> h) a liquid cooling aid, wherein a portion of the outer surfaces of the tubes are arranged and adapted to be contacted with the liquid cooling aid, with the portion being at least 50 %, preferably at least 70 %, more preferably at least 80 %, and further preferably at least 90 % of the length of the additional longitudinal portion;

and wherein,
an average thickness of any layer comprising the particulated solid material present in the outlet volume, the inlet volume, or both, is less than 10 %, preferably less than 5 %, and more preferably less than 3 % of the average length of the further longitudinal portions.

|2| The reactor assembly according to embodiment |1|, wherein at least one or all of the following applies:

a) the mass percentage of the particulated solid material present in the first longitudinal portion is less than 5 wt-%, preferably less than 3 wt-%, and more preferably less than 1 wt-%, based on the total mass of the particulated solid material contained in the tube;
b) the mass percentage of the first solid catalyst contained in the first longitudinal portion is less than 5 wt-%, preferably less than 3 wt-%, and more preferably less than 1 wt-%, based on the total mass of the first solid catalyst contained in the tube.

For embodiment |2|, all possible combination of the features a. and b. are preferred aspects of the embodiment. These combinations are e.g., a; b; a, b.

|3| The reactor assembly according to any of the preceding embodiments, wherein at least 30 %, preferably at least 50 %, more preferably at least 80 %, and further preferably at least 90 % of the length of the further longitudinal portions is embedded in the further metal plate.

|4| The reactor assembly according to any of the preceding embodiments, wherein at least 30 %, preferably at least 50 %, more preferably at least 80 %, and further preferably at least 90 % of the length of the first longitudinal portions is embedded in the first metal plate.

|5| The reactor assembly according to any of the preceding embodiments, wherein the additional longitudinal portion has at least twice, preferably at least 10 times, and more preferably at least 30 times the length of the further longitudinal portion. In many cases, the length of the additional longitudinal portion does not exceed 100 times the length of the further longitudinal portion.

|6| The reactor assembly according to any of the preceding embodiments, wherein the additional longitudinal portion has at least twice, preferably at least 10 times, and more preferably at least 30 times the length of the first longitudinal portion. In many cases, the length of the additional longitudinal portion does not exceed 110 times the length of the first longitudinal portion.

|7| The reactor assembly according to any of the preceding embodiments, wherein the further longitudinal portions are in thermal contact with the further metal plate.

|8| The reactor assembly according to any of the preceding embodiments wherein the further metal plate is in thermal contact with the liquid cooling aid.

|9| The reactor assembly according to any of the preceding embodiments, wherein at least one or all of the following applies:

a) the mass percentage of the first solid catalyst contained in the additional longitudinal portion is more than 50 wt-%, preferably more than 70 wt-%, and more preferably more than 85 wt-%, based on the total mass of the first solid catalyst contained in the tube;
b) the ratio of a mass of the solid first catalyst to a mass of the particulated solid material, both contained in the further longitudinal portion, is 1:$x$, where $x$ is at least 10, preferably at least 100, and more preferably at least 500. Here x represents the mass of the particulated solid material.

For embodiment |9|, all possible combination of the features a. and b. are preferred aspects of the embodiment. These combinations are e.g., a; b; a, b.

|10| The reactor assembly according to any of the preceding embodiments, wherein the particulated solid material comprises one or more components selected from the group consisting of metals and metal oxides, preferably a mixture of at least two components selected from the aforementioned group.

|11| The reactor assembly according to any of the preceding embodiments, wherein the particulated solid material is selected from the group consisting of aluminium, copper, steel, silicon oxide, aluminium oxide, zircon oxide, sandstone, granite, or a combination of two or more thereof, such as ceramics.

|12| The reactor assembly according to any of the preceding embodiments, wherein the particulated solid material has at least one or all of the following physical properties:

a) thermal conductivity in the range of 0.01 W/m/°C to 500 W/m/°C, preferably in the range of 0.05 W/m/°C to 450 W/m/°C, and more preferably in the range of 0.1 W/m/°C to 400 W/m/°C;
b) specific geometric surface area in the range of 20 m$^2$/m$^3$ to 2000 m$^2$/m$^3$, more preferably in the range of 100 m$^2$/m$^3$ to 1500 m$^2$/m$^3$, and further preferably in the range of 800 m$^2$/m$^3$ to 1200 m$^2$/m$^3$;
c) free volume in the range of 24 % to 99 %, preferably in the range of 30 % to 99 %, and further preferably in the range of 35 % to 99 %; or
d) particle size distribution in the range of 0.01 mm to 100 mm, preferably in the range of 0.05 mm to 50 mm, and more preferably in the range of 0.1 mm to 30 mm.

For embodiment |12|, all possible combination of the features a. to d. are preferred aspects of the embodiment. These combinations are e.g., a; b; c; d; a, b; a, c; a, d; b, c; b, d; c, d; a, b, c; a, b, d; a, c, d; b, c, d; a, b, c, d.

|13| The reactor assembly according to any of the preceding embodiments, wherein at least one or all of the following properties apply to at least 50 %, preferably at least 80 %, and more preferably at least 95 % of the tubes:

> a) the tube length is in the range of 1000 mm to 15000 mm, preferably in the range of 2000 mm to 9000 mm, and more preferably in the range of 3000 mm to 6000 mm;
> b) the diameter of the inner surface in the range of 10 mm to 100 mm, preferably in the range of 12 mm to 50 mm, or more preferably in the range of 18 mm to 46 mm; or
> c) the tubes are arranged parallel to each other.

For embodiment |13|, all possible combination of the features a. to c. are preferred aspects of the embodiment. These combinations are e.g., a; b; c; a, b; a, c; b, c; a, b, c.

|14| The reactor assembly according to any of the preceding embodiments, wherein the liquid cooling aid has a temperature in the range of 150 °C to 420 °C, preferably in the range of 200 °C to 370 °C, and more preferably in the range of 230 °C to 360 °C.

|15| The reactor assembly according to any of the preceding embodiments, wherein the first metal plate, the further metal plate, or both, have independently from each other a thickness in the range of 10 mm to 300 mm, preferably in the range of 15 mm to 180 mm, and more preferably in the range of 20 mm to 160 mm. In one aspect of this embodiment, it is preferred that the first longitudinal portion has a length in the range of 0.5 to 4, preferably in the range of 0.6 to 3.2, and more preferably in the range of 0.7 to 2.9 times the thickness of the first metal plate. In another aspect of this embodiment it is preferred that the further longitudinal portion has a length in the range of 0.5 to 4, preferably in the range of 0.6 to 3.2, and more preferably in the range of 0.7 to 2.9 times the thickness of the further metal plate.

|16| The reactor assembly according to any of the preceding embodiments wherein the liquid cooling aid flows into the first reactor through at least one inflow-opening, and out of the first reactor through at least one outflow-opening.

|17| The reactor assembly according to any of the preceding embodiments, wherein the first longitudinal portion, the additional longitudinal portion, and the further longitudinal portion cover at least 50 %, preferably at least 65 %, more preferably at least 80 %, and further preferably at least 90 % of the tube length.

|18| The reactor assembly according to any of the preceding embodiments, wherein the first solid catalyst is selected from the group consisting of mixed metal oxides or a combination of at least two thereof.

|19| The reactor assembly according to any of the preceding embodiments, wherein the reactor assembly further comprises a second reactor,

> a. wherein the second reactor is located upstream of the first reactor;
> b. wherein the second reactor comprises

>> i. a second inlet,
>> ii. a second outlet that is in fluid communication with the first inlet of the first reactor,
>> iii. a second catalytic reaction zone that is located between the second inlet and the second outlet,

>>> a.) wherein the second reaction zone comprises a second solid catalyst,
>>> b.) wherein the second solid catalyst is arranged and adapted for partially oxidising a gaseous composition having an aldehyde precursor content of at least 2 vol-%, preferably at least 2.5 vol-%, and more preferably at least 3 vol-%, to an aldehyde with a selectivity of at least 70 %, preferably at least 75 %, and more prefer-ably at least 80 %, based on the unsaturated hydrocarbon content.

|20| The reactor assembly according to the preceding embodiment |19|, wherein the aldehyde precursor is selected from the group consisting of isobutylene, methyl tert-butyl ether, and tertiary butyl alcohol, or a combination of at least two thereof.

|21| The reactor assembly according to any of the preceding embodiments |19| to |20|, wherein the second solid catalyst is selected from the group consisting of mixed metal oxides or a combination of at least two thereof.

|22| The reactor assembly according to any of the preceding embodiments, wherein the assembly reactor further comprises a third reactor,

> a. wherein the third reactor is located downstream of the first reactor;
> b. wherein the third reactor comprises

i. a third inlet that is in fluid communication with the first outlet of the first reactor,
ii. a third outlet,
iii. a third catalytic zone that is located between the third inlet and the third outlet,

> a.) wherein the third catalytic zone comprises a third catalyst,
> b.) wherein the third catalyst is arranged and adapted for esterifying a composition, preferably a liquid composition, having a C3 - C6 carboxylic acid component of at least 30 vol-%, preferably at least 45 vol-%, and more preferably at least 55 vol-%, to an alkyl ester, preferably an alk-alkyl ester, more preferably a meth-alkyl ester and further preferred methyl methacrylate.

|23| The reactor assembly according to the preceding embodiment |22|, wherein the third catalyst is selected from the group consisting of an ion exchanger or a Lewis acid, more preferably an acidic ion exchange resin or acids capable of catalysing esterification, or a combination of at least two thereof.

|24| The reactor assembly according to any of the preceding claims, wherein the reactor assembly comprises a gas cooler connected to the first outlet.

|25| A method for producing a product, preferably a polymer or an unsaturated alkyl carboxylic compound, more preferably an unsaturated alkyl carboxylic acid or an unsaturated alkyl ester, further preferably an alk alkyl ester, even more preferably a meth alkyl ester, and in particular preferred methyl methacrylate, wherein the method comprises the following process steps:

a) providing a gaseous feed stream, wherein the feed stream comprises an $C_3$ - $C_6$ aldehyde;
b) partially oxidising the $C_3$ - $C_6$ aldehyde in the feed stream in the presence of a solid catalyst to obtain a first product stream comprising a partial oxidation product, wherein the partial oxidation is performed along an additional longitudinal portion, and
c) cooling the first product stream along a further longitudinal portion, preferably to a temperature below 350 °C, more preferably below 340 °C, further preferably below 330 °C and even more preferably below 320 °C, wherein

(i) the further longitudinal portion comprises a particulated solid material,
(ii) the temperature of the product stream in the further longitudinal portion decreases at a rate $R_p$; and

wherein
$R_p$ is larger than a rate of temperature decrease $R_e$, wherein the rate $R_e$ is for a scenario where the further longitudinal portion does not comprise the particulated solid material.

|26| The method according to embodiment |25|, wherein $R_p$ is at least 2 times larger, preferably at least 5 times larger, more preferably at least 10 time larger, and further preferably at least 21 times larger than $R_e$.

|27| The method according to any of the embodiments |25| to |26|, wherein the rate $R_p$ is in the range of 0.1 °C/cm to 5 °C/cm, preferably in the range of 0.2 °C/cm to 2.5 °C/cm, and more preferably in the range of 0.6 °C/cm to 1.6 °C/cm.

|28| The method according to any of the embodiments |25| to |27|, wherein the temperature of the first product stream that exits the further longitudinal portion is below the autoxidation temperature of the first product stream.

|29| The method according to any of the embodiments |25| to|28|, wherein the temperature of the first product stream that exits the further longitudinal portion is in the range of > 230 °C to 360 °C, preferably in the range of 270 °C to 335 °C, and more preferably in the range of 295 °C to 320 °C.

|30| The method according to any of the embodiments |25| to |29|, wherein the temperature of the first product stream that enters the further longitudinal portion is in the range of 250 °C to 400 °C, preferably in the range of 260 °C to 390 °C, and more preferably in the range of 280 °C to 380 °C.

|31| The method according to any of the embodiments |25| to |30|, wherein the temperature of the feed stream that enters the first longitudinal portion is in the range of 180 °C to 300 °C, preferably in the range of 200 °C to 280 °C, and more preferably in the range of 220 °C to 260 °C.

|32| The method according to any of the embodiments |25| to |31|, wherein the pressure decrease between the inlet volume and the outlet volume is in the range of 100 mbar to 350 mbar, preferably in the range of 130 mbar to 300 mbar, and more preferably in the range of 150 mbar to 280 mbar.

|33| The method according to any of the embodiments |25| to |32|, wherein the average velocity of the first product stream in the further longitudinal portion is in the range of 0.5 m/s to 12 m/s, preferably in the range in the range of 0.8 m/s to 11.5 m/s, and more preferably in the range of 1.0 m/s to 11 m/s.

|34| The method according to any of the embodiments |25| to |32|, wherein the product stream that exits the first outlet is fed into a gas cooler and wherein the temperature of the product stream that exits the gas cooler is in the range of 160 °C to 280 °C, preferably in the range of 190 °C to 270 °C, and more preferably in the range of 200 °C to 260 °C.

|35| The method according to any of the embodiments |25| to |33|, wherein the $C_3$ - $C_6$ aldehyde is present in the first product stream in an amount in the range of 0.1 % to 10 %, preferably in the range of 0.3 % to 7 %, more preferably in the range of 0.5 % to 5 %, and further preferably in the range of 0.7 % to 4 %, based on the amount of $C_3$ - $C_6$ aldehyde in the feed stream.

|36| The method according to any of the embodiments |25| to |35|, wherein the $C_3$ - $C_6$ aldehyde is acrolein, preferably methacrolein.

|37| The method according to any of the embodiments |25| to |36|, wherein the product is methacrylic acid or methyl methacrylate.

|38| The method according to any of the embodiments |25| to |37|, wherein the feed stream comprises

a) at least 2 mol-%, preferably at least 2.5 mol-%, and more preferably at least 3 mol-% unsaturated $C_3$ - $C_6$ aldehyde, preferably methacrolein;

b) mol-% water that is at least 2 times larger, preferably at least 3 times larger, more preferably at least 4 times larger than the vol-% $C_3$ - $C_6$ aldehyde;

c) mol-% oxygen that is 2 to 3.5 times larger, preferably 2.2 to 3.3 times larger, more preferably 2.4 to 3.2 times larger, than the vol-% $C_3$ - $C_6$ aldehyde;

d) less than 3 mol-%, preferably less than 1.5 mol-%, more preferably less than 1 mol-% unsaturated $C_3$ - $C_6$ carboxylic acid, preferably methacrylic acid;

wherein the sum of the vol-% of the components of the feed stream being equal to 100 vol-%.

|39| The method according to any of the embodiments |25| to |38|, wherein the first product stream further comprises as components

a) not more than 10 vol-%, preferably not more than 18 vol-%, and more preferably not more than 20 vol-%, water;

b) not more than 5 vol-%, preferably not more than 7 vol-%, and more preferably not more than 10 vol-%, oxygen;

c) at least 0.5 vol-%, preferably at least 1.0 vol-%, more preferably at least 1.5 vol-%, and further preferably at least 1.8 vol-% unsaturated $C_3$ - $C_6$ carboxylic acid, preferably methacrylic acid;

d) not more than 2 vol-%, preferably not more than 1.5 vol-%, more preferably not more than 1.2 vol-%, and further preferably not more than 0.6 vol-% acetic acid;

e) not more than 0.9 vol-%, preferably not more than 1 vol-%, and more preferably not more than 1.1 vol-%, (meth)acrolein;

wherein the sum of the vol-% of the components of the first product stream being equal to 100 vol-%.

|40| The method according to any of the embodiments |25| to |39|, wherein the additional longitudinal portion and the further longitudinal portion are located in a tube.

|41| The method according to any of the embodiments |25| to |40|, wherein the further longitudinal portion is adjacent to the additional longitudinal portion.

|42| The method according to any of the embodiments |25| to |41|, wherein the first product stream is subjected to an esterification reaction in order to obtain the product in the form of an unsaturated alkyl ester, preferably methyl methacrylate.

|43| The method according to embodiment |42|, wherein the unsaturated alkyl ester is subjected to at least one purification step.

|44| The method according to any of the embodiments |42| and |43|, wherein the unsaturated alkyl ester is subjected to a polymerisation reaction in order to obtain the product in form of a polymer, preferably poly methyl methacrylate.

|45| A use of an unsaturated alkyl ester obtainable by a method according to embodiments |42| to |44| for making a polymer.

|46| A polymer obtainable by a method according to embodiment |45|.

|47| A use of the polymer according to embodiment |46| in an item selected from the group consisting of shaped articles, moulding materials, films, sheets, granulates, composites, foams, fibres, lubricants, adhesives, thickening agents, suspending agents, flocculants, resins, plastics, coatings, contact lenses, construction materials, absorbent materials, pharmaceuticals, materials for controlled release of active substances, powders, or a combination of at least two thereof.

## Detailed description of the invention

In this section selected aspects of preferred embodiments of the invention are discussed in more detail.

The first reactor

As is well-known to a person skilled in the art, tube bundle reactors are particularly suitable for partially oxidising a gaseous feed stream on a catalyst to obtain a product stream. Such reactors are commercially available, *e.g.*, from Deggendorfer Werft, Germany, or from IHI Corporation, Japan. Due to their ubiquitous use and commercial availability, operating a tube bundle reactor is generally well within the knowledge of a person skilled in the art.

In the context of the present invention, the term "*feed stream*" refers to the gaseous mixture of chemical compounds and single chemical elements prior to the start of partial oxidation, preferably prior to contact with a catalyst. In the context of the present invention, the term "*product stream*", or similar phrases, should be understood to mean that the feed stream has come into contact with a catalyst, and that partial oxidation of at least one of the comprising components of the feed stream has started.

A first aspect of the invention is a reactor assembly that comprises a first reactor for the partial oxidation of a feed stream to a product stream. The first reactor is a commercially available tube bundle reactor that has been modified with the purpose of cooling the product stream in order to suppress further oxidation (also called "*autoxidation*").

Autoxidation is an incomplete and uncontrolled oxidation which produces carbon residues which tend to clog the reactor. As a result, the yield of the reactor is decreased. Autoxidation strongly depends on the pressure and the temperature, and even more on the concentration of the main component in the product stream susceptible to autoxidation (*e.g.*, (meth)acrolein). Moreover, the $O_2$ concentration and residence time of the reactants also impacts autoxidation. At concentrations above 3 vol-%, (meth)acrolein autoxidation has been observed at 240 °C or higher. At 1 vol-%, (meth)acrolein autoxidation has been observed at 315 °C or higher.

In another aspect of the invention, the first reactor comprises a first inlet that allows a feed stream to flow into, and a first outlet that allows a product stream to flow out of, the first reactor.

According to a further aspect of the invention, located between the first inlet and the first outlet is a plurality of tubes, with every tube comprising an inlet-end that allows the feed stream to flow into the tube, and an outlet-end that allows the product stream to flow out of the tube. In another aspect of the invention, every tube has an outer surface, as well as an inner surface that confines an inner volume of the tube.

According to a further aspect of the invention, at least a portion of the outer surfaces of the tubes are immersed in, and in thermal contact with, a liquid cooling aid. The phrase "*in thermal contact*" should be understood to mean that heat can flow between any number of components that are in thermal contact with each other. It is preferred that the any number of components that are "*in thermal contact*" with each other also touch each other.

In one aspect of the invention, at least 50 % of the tubes further comprise an additional longitudinal portion and a further longitudinal portion, with the latter downstream to the former. It is preferred in this aspect that the inner volume of the additional longitudinal portion is at least partially filled with a first solid catalyst suitable for the partial oxidation of the feed stream to the product stream. It is in particular preferred in this aspect that the inner volume of the further longitudinal portion is at least partially filled with an inert particulated solid material that is arranged and adapted to dissipate heat. In this aspect it is also preferred that the inner volume of the additional longitudinal portion is at least partially filled with a mixture of the first solid catalyst and the particulated solid material.

The term "*inert*" should be understood to mean that the presence of the particulated solid material does not lead to any measurable change in the composition of the feed stream or the product stream.

The phrase "*dissipate heat*" should be taken to mean that the product stream is in thermal contact with the particulated solid material, and that this leads to the product stream being cooled more rapidly compared to a scenario wherein the product stream is not in contact with the particulated solid material. *E.g.*, a temperature of the product stream in a centre of the further longitudinal portion is higher than a temperature of the product stream that flows along a side wall of the further longitudinal portion. The particulated solid material dissipates heat by transferring heat from the centre of the further longitudinal portion to the side wall of the further longitudinal portion.

In a further aspect of the invention, it is preferred that at least 50 % of the tubes comprise a first longitudinal portion. In this aspect it is further preferred that the first longitudinal portion is upstream the additional longitudinal portion. While the first longitudinal portion can be at least partially filled with the first solid catalyst, it is more preferred that the first longitudinal portion does not contain any first solid catalyst. While the first longitudinal portion can be at least partially filled with the particulated solid material, it is more preferred that the first longitudinal portion does not contain any particulated solid material. It is particularly preferred that the first longitudinal portion does not contain any first solid catalyst or any particulated solid material.

In the context of the present invention, a longitudinal portion comprises two end points. The two end points are characterised as the positions where a ratio of a mass of the first solid catalyst to a mass of the particulated solid material changes by more than 30 %. It should be noted that two adjacent longitudinal portions will share an end point. As an example, consider a tube that has the first longitudinal portion, the additional longitudinal portion, and the further longitudinal portion. Furthermore, the first longitudinal portion and the additional longitudinal portion are adjacent to each other, while the additional longitudinal portion and the further longitudinal portion are adjacent to each other. Next, consider the two example set-ups:

> i. the first longitudinal portion is empty, the additional longitudinal portion is filled with only the first solid catalyst, and the further longitudinal portion is filled with only the particulated solid material;
>
> ii. the first longitudinal portion is filled with only the particulated solid material, the additional longitudinal portion is filled with a mixture of the first solid catalyst and the particulated solid material, and the further longitudinal portion is filled with only the particulated solid material.

In both these set-ups it is clear that the ratio of the mass of the of the first solid catalyst to the mass of the particulated solid material changes by more than 20 % between two adjacent longitudinal portions.

In one aspect of the invention, the first reactor comprises a first metal plate. In this aspect it is preferred that the first metal plate is made from stainless steel or carbon steel. In another aspect of the invention, the first reactor comprises a further metal plate. In this aspect it is preferred that the further metal plate is made from stainless steel or carbon steel.

According to an aspect of the invention, a further metal plate is arranged and adapted to allow the further longitudinal portion to be positioned in the further metal plate. In this aspect it is preferred that the further longitudinal portion passes through the further metal plate: *e.g.,* a length of the further longitudinal portion is longer than a thickness of the further metal plate; *e.g.*, the further longitudinal portion extends through the further metal plate into the outlet volume. However, it is more preferred that the outlet-end of the tube is flush with a surface of the further metal plate, further preferable a surface of the further metal plate that does not touch the liquid cooling aid. The preferred embodiments disclosed in this paragraph is also preferred, *mutatis mutandis*, for the aspect of the invention wherein the first metal plate is arranged and adapted to allow the first longitudinal portion to be positioned in the first metal plate. It is also preferred that the metal plate is made from stainless steel or carbon steel.

In another aspect of the invention, the first reactor further comprises two volumes associated with the first inlet and the first outlet: the inlet-ends of the tubes and the first inlet confine an inlet volume, while the outlet-ends of the tubes and the first outlet confine an outlet volume. According to an aspect of the invention, the first inlet, the inlet volume, the tubes, the outlet volume, and the first outlet, are in fluid communication with each other.

The term "*fluid*" should be understood to mean any element, compound, or physical matter that is in the gaseous or liquid thermodynamic phases of matter. A "*fluid*" can be at least one of a liquid, a gas, a vapour, a supercritical fluid, or any other fluid. *E.g.,* water vapour, the gaseous feed stream, and salt dissolved in water are considered as examples of a "*fluid*".

The phrase *"in fluid communication"* should be understood to mean that a fluid can flow from a first component of a first device to any other component of the first device or a further device that is *"in fluid communication"* with the first component of the first device. *E.g.*, a first partial oxidation reactor and a third esterification reactor are *"in fluid communication"* if a fluid can flow from the first reactor to the third reactor, or vice versa. *E.g.*, a first partial oxidation reactor contains a volume between the tubes, with this volume filled with a liquid cooling aid. This volume is not *"in fluid communication"* with, *e.g.,* the first outlet of the first reactor, as a fluid cannot flow from this volume to the first outlet.

It should be noted that components that are *"in fluid communication"* with each other does not imply that the components have to be adjacent to each other. *E.g.*, between a first partial oxidation reactor and a third esterification reactor that are "*in fluid communication*", there can be *e.g.*, devices used for cooling and purifying the product stream produced in the first reactor, prior to the product stream flowing into the third reactor.

<u>The tubes</u>

In the context of the present invention, the term "*tube*" refers to any number of components of a device capable of directing the streamlines of a fluid. It is preferred that the "*tube*" has an opening at both ends. It is also preferred that a "*tube*" has an enclosed inner volume through which a fluid can flow, that the inner volume can be filled with a material such as a solid catalyst, or both.

It is not a requirement that the components of a "*tube*" should be adjacent to each other, only that the components should be in fluid communication. *E.g.*, "*a tube*" can refer to two pipes and a further metal plate placed between the pipes, wherein the further metal plate has a hollow that extends through the further metal plate. A fluid can thus flow through one pipe, via the hollow in the further metal plate, into the second pipe.

With regards to a number of the aspects of the invention related to the tubes, the following should be noted: while there is no particular limitation to the shape of the tubes, straight tubes are preferred. It is also preferred that the tubes have a circular cross-section. It is also preferred that the additional longitudinal portion and the further longitudinal portion are adjacent to each other. It is further preferred that the first longitudinal portion is adjacent to the additional longitudinal portion.

It is preferred that the length of a first longitudinal portion does not differ by more than 20 %, preferably by not more than 15 %, and more preferably by not more than 10 % from the average length of the first longitudinal portions. It is preferred that the length of an additional longitudinal portion does not differ by more than 18 %, preferably by not more than 12 %, and more preferably by not more than 6 % from the average length of the additional longitudinal portions. It is preferred that the length of a further longitudinal portion does not differ by more than 20 %, preferably by not more than 15 %, and more preferably by not more than 10 % from the average length of the further longitudinal portions.

It is preferred in an aspect of the present invention that the length of the additional longitudinal portion is in the range of 0.5 to 0.98, preferably in the range of 0.6 to 0.97, and further preferably in the range of 0.8 to 0.95 times the tube length. It is preferred that the length of the further longitudinal portion is smaller than the length of the additional longitudinal portion. It is more preferred that the length of the further longitudinal portion is in the range of 40 mm to 300 mm, further preferably in the range of 60 mm to 280 mm, and even further preferably in the range of 90 mm to 250 mm. It is preferred that the length of the first longitudinal portion is smaller than the length of the additional longitudinal portion. It is more preferred that the length of the first longitudinal portion is in the range of 40 mm to 200 mm, further preferably in the range of 60 mm to 180 mm, and even further preferably in the range of 90 mm to 150 mm.

The outlet volume

It is possible that at least one layer comprising particulated solid material can be present in the inlet volume, the outlet volume, or both. This layer can be *e.g.,* in the form of a sheet with uniform thickness that covers the outlet-ends of the tubes and an outer surface of the further metal plate. The sheet can be subdivided into sections, wherein every section is filled with the particulated solid material in the shape of, for example, weaves. *E.g.*, in the form of heaps of particulated solid material that cover only the outlet-ends. Here the outer surface of the further metal plate is defined as a surface of the further metal plate that does not touch the liquid cooling aid.

It is preferred that the average thickness of the layer in the outlet volume is less than 200 mm, more preferably less than 100 mm, and further preferably less than 50 mm. It is even further preferred that the outlet volume does not contain any particulated solid material. It is preferred that the average thickness of the layer in the inlet volume is less than 200 mm, more preferably less than 100 mm, and further preferably less than 50 mm. It is even further preferred that the inlet volume does not contain any particulated solid material. It is particularly preferred that both the inlet volume and the outlet volume do not contain any particulated solid material.

The first catalyst

According to an aspect of the invention, the additional longitudinal portion is filled with a first solid catalyst that is capable of partially oxidising the feed stream. Many solid catalysts suitable for this purpose are generally known to the person skilled in the art. Amongst these, solid catalysts suitable for the partial oxidation of a $C_3$ - $C_6$ aldehyde to a carboxylic acid are preferred. More preferred are solid catalysts comprising a metal oxide, and further preferred are catalysts comprising mixed metal oxides.

Examples of suitable catalysts include those described in detail in WO 2008/145418 A1, WO 2004/073857 A1, WO 2019/141203 A1 and US 5,583,084, with WO 2019/141203 A1 being preferred. It is also preferred not to limit the choice of suitable catalyst to a single catalyst, and a mixture comprising any number of suitable catalysts can be used.

The particulated solid material

According to an aspect of the invention, the further longitudinal portion is filled with a particulated solid material that is arranged and adapted to dissipate heat. The choice of particulated solid material is not especially limited, with many suitable examples generally known to a person skilled in the art. These examples include oxides, sulphides, nitrides, carbides, carbonates and silicates of alkali metals, alkaline earth metals and transition metals, as well as the metals, semi-metals and non-metals of the main groups III, IV, and V of the periodic table, and their mixtures.

Examples of preferred choices include magnesium oxide, calcium oxide, $\alpha$-alumina, $\gamma$-alumina, silicon dioxide, titanium dioxide, zinc oxide, silicon nitride, aluminium silicates, ceramic materials, common refractory materials and glasses. Also preferred choices include silicon carbide, bentonite, steatite, mullite, kyanite, pumice stone, kieselguhr and kaolin or a combination of two or more thereof. Particularly preferred are $\alpha$-alumina or $\gamma$-alumina, or both.

It is further preferred to select the particulated solid material from a group of materials (chemical elements and their compounds) that have thermal conductivities that fall into either of two ranges. For the first range, it is preferred if the thermal conductivity is in the range of 0.01 W/m/°C to 8.5 W/m/°C, more preferably in the range of 0.05 W/m/°C to 5 W/m/°C, and further preferably in the range of 0.1 W/m/°C to 2 W/m/°C. For the second range it is preferred if the thermal conductivity is in the range of 10 W/m/°C to 150 W/m/°C, more preferably in the range of 11 W/m/°C to 70 W/m/°C, and further preferably in the range of 12 W/m/°C to 25 W/m/°C.

It is preferred that materials in the first thermal conductivity range have a lower chemical reactivity than those in the second range. Examples of particulated solid material that fall into the first range are inorganic materials such as sand, sandstone, and granite. Particulated solid materials that fall into the second range are generally metals, with examples being steel, aluminium, and copper. If the particulated solid material is steel, stainless steel is preferred.

It is preferred not to limit the choice of particulated solid material to a single material, but to a mixture that can comprise any number of the above-mentioned elements and compounds. It is more preferred to combine one or more particulated solid material from the first thermal conductivity range with one or more particulated solid materials from the second thermal conductivity range.

In one aspect of the invention, there is no limitation geometric shape for the individual particles that make up the particulated solid material. *E.g.*, the particulated solid material can have, to a first approximation, one or more of any of the following shapes: spheres, rings, rods, tablets, brushes, grids, hollow cylinders, and foams, or a combination of two or more of the above. *E.g.*, the particulated solid material can also be in the form of one or more weaves, webs, or knits. It is, however, preferred that the particle size distribution of the particulated solid material is determined by at least one dimension of the tubes, more preferably the diameters of the inner surfaces of the tubes.

It is preferred that the particulated solid material should be gas-permeably arranged and adapted. The phrase "*gas-permeably arranged and adapted*" should be understood to mean that the particulated solid material can be porous, or more preferably, that the particulated solid material contains volumes through which the product stream can flow. These volumes are preferably located between, or in, the individual particles that make up the particulated solid material. They may occur due to the geometric shape of the individual particles. *E.g.,* if the individual particles are spheres of similar size, then small volumes will be present between the spheres, even if the spheres are touching each other. *E.g.,* if the individual particles are hollow cylinders of similar size, then the product stream can flow through the inner volumes of the cylinders.

The particulated solid material is commercially available from *e.g.*, Vereinigte Füllkörper-Fabriken GmbH & Co. KG, Germany, and Raschig GmbH, Germany.

## The liquid cooling aid

According to an aspect of the invention, at least a portion of the outer surfaces of the tubes are immersed in, and in thermal contact with, a liquid cooling aid. In this aspect it is preferred that the outer surfaces of at least 50 %, more preferably at least 80 %, and further preferably at least 95 % of the tubes are in thermal contact with the liquid cooling aid. The phrase "*immersed in*" should be understood to mean that the liquid cooling aid can flow through the volumes between the tubes.

The liquid cooling aid, functioning as a heat transfer aid, provides a mechanism for regulating the temperature of the product stream in the tubes, preferably the temperature of the catalyst, the product stream, or both, in the additional longitudinal portion. The phrase "*cooling aid*" should therefore not be taken to mean that the liquid cooling aid is particularly used to cool the tubes. If necessary, the liquid cooling aid can also be used to heat the tubes. The choice of liquid cooling aid is not particularly limited, with many options generally known to the person skilled in the art. A suitable liquid cooling aid in the form of a salt bath is commercially available from *e.g.,* HEF Durferrit GmbH, Germany. A suitable liquid cooling aid in the form of an oil is commercially available as *e.g.*, Marlotherm from Sasol GmbH, Germany.

In another aspect of the invention, it is preferred that the further metal plate is in thermal contact with the liquid cooling aid, thereby allowing the temperature of the further metal plate to be regulated by the liquid cooling aid. The temperature of the further metal plate can optionally be regulated independently from the temperature of the liquid cooling aid. However, such independent temperature regulation of the further metal plate generally adds unnecessary complexity to the first reactor without adding any benefit to either productivity or safety, while often also requiring additional maintenance.

In one aspect of the invention, it is preferred that the first reactor further comprises at least one inflow-opening through which the liquid cooling aid can flow into the first reactor, as well as at least one outflow-opening through which the liquid cooling aid can flow out of the reactor. This allows for a circulation of the liquid cooling aid through the first reactor.

In this aspect it is preferred that the at least one inflow-opening is located closer to the further metal plate than to the first metal plate, and that the at least one outflow-opening is located closer to the first metal plate than to the further metal plate. However, in this aspect it is particularly preferred that the at least one inflow-opening is located closer to the first metal plate than to the further metal plate, and that the at least one outflow-opening is located closer to the further metal plate than to the first metal plate.

In a further aspect of the invention, it is preferred that the temperature of the liquid cooling aid is regulated outside of the reactor. This can be done using any heating / cooling device generally known to a person skilled in the art and commercially available by, *e.g.*, Deggendorfer Werft, Germany.

The second reactor

According to an aspect of the invention, the reactor assembly optionally comprises a second reactor that is upstream, and in fluid communication with, the first reactor. The second reactor is used for partially oxidising an aldehyde precursor to a $C_3$ - $C_6$ aldehyde, with this $C_3$ - $C_6$ aldehyde being a comprising component of the feed stream that flows into the first reactor.

The preferred $C_3$ - $C_6$ aldehyde that is produced is preferably a $C_3$ - $C_6$ alkacrolein, and more preferably methacrolein. Further details of the process for producing the $C_3$ - $C_6$ aldehyde is described in, *e.g.,* WO 2008/145418 A1 and EP 1 995 232 B1.

It is preferred that the second reactor is also a tube bundle reactor. Thus, any of the embodiments or aspects of the first reactor, preferred or otherwise, or any of the tube bundle reactors disclosed in, *e.g.,* DE 30 42 468 A1, DE 100 47 693 A1, and DE 198 06 810 A1 also apply to the second reactor.

The third reactor

According to an aspect of the invention, the reactor assembly optionally comprises a third reactor that is downstream, and in fluid communication with, the first reactor. The third reactor is adapted and arranged for esterifying the product stream produced in the first reactor on a third catalyst to obtain an ester.

It is preferred that the product stream comprises a $C_3$ - $C_6$ carboxylic acid that is esterified to obtain an alkyl ester, preferably an alk-alkyl ester, more preferably a meth-alkyl ester and further preferred methyl methacrylate.

The third reactor is not particularly limited, and any reactor suitable for esterification can be used. However, reactors suitable for liquid phase esterification is preferred. More preferred are reactors suitable for the esterification of a $C_3$ - $C_6$ carboxylic acid to form methyl methacrylate. Further details of the esterification process in relation to the present invention can be found in, *e.g.,* WO 2008/145418 A1 and EP 1 995 232 B1.

Producing the product

If the product is an unsaturated alkyl carboxylic acid, it is preferred to produce the product using any of the embodiments of the first reactor as disclosed in a previous section. If the product is an unsaturated alkyl ester, it preferred to produce the product in an esterification step. It is even further preferred to perform this esterification step using any of the embodiments of the third reactor as disclosed in a previous section.

If the product is a polymer, it is preferred to produce the product by subjecting an unsaturated alkyl ester to a polymerisation reaction in order to obtain the product in the form of a polymer, preferably polymethyl methacrylate. The polymerisation is not particularly limited and can be carried out by any method generally known to the skilled person and appearing suitable, for example as described in US 5,292,797, US 4,562,234, US 5,773,505, US 5,612,417, US 4,952455, US 4,948,668, US 4,239,671. Preferred polymerisation methods are radical polymerisation, initiated by initiators which decompose into radicals under the polymerisation conditions, whereby the polymerisation is preferably a solution or an emulsion polymerisation, preferably an aqueous solution polymerisation.

Further details regarding the methods and devices needed to either obtain the product in the form of an unsaturated alkyl ester or a polymer, or using the unsaturated alkyl ester to produce the polymer, is discussed in, *e.g.,* EP 1 995 232 B1.

The present invention preferably pertains to a device and method for cooling a product stream that is produced by the partial oxidation of a $C_3$ - $C_6$ aldehyde. Further details of the process for producing the feed stream comprising the $C_3$ - $C_6$ aldehyde, the partial oxidation steps including the partial oxidation to the $C_3$ - $C_6$ aldehyde, the esterification step, and any additional steps that may be required to produce the polymer is disclosed in, *e.g.,* WO 2008/145418 A1 and EP 1 995 232 B1.

Temperature regulation

The partial oxidation of a gaseous feed stream to obtain a first product stream should proceed in a controlled manner, and according to an aspect of the invention, generally takes place in an additional longitudinal portion that comprises a solid catalyst suitable for partial oxidation. It is preferred that the autoxidation that could occur outside the additional longitudinal portion should be suppressed.

One aspect of the invention is cooling the first product stream when the product stream is not in contact with the solid catalyst. It is preferred that the cooling takes place in a further longitudinal portion. It is furthermore preferred that the cooling is done at a sufficiently fast rate so that the requirement $R_p > R_e$ is satisfied.

The rate of temperature decrease $R_p$ is defined as the rate when the further longitudinal portion comprises particulated solid material. The rate of temperature decrease $R_e$ is defined as the rate when the further longitudinal portion does not contain particulated solid material.

A rate of temperature decrease, $R$, is defined as

$$R = \frac{T(x_1) - T(x_2)}{\Delta x},$$

where $T$ is the temperature, $\Delta x = x_2 - x_1$, and $x_1$ and $x_2$ are two points in space. The coordinate $x$-axis is arranged parallel to a line that traces the centre of the further longitudinal portion, with $x_2$ downstream to $x_1$. Thus $x_2 > x_1$ and $\Delta x > 0$. As the further longitudinal portion can be located in *e.g.,* a straight tube, or a tube in the shape of an "S", the variable $\Delta x$ refers to the distance that a fluid parcel needs to travel in order to flow through the further longitudinal portion, assuming the flow is laminar. Note that $R$ is defined in such a way that $R > 0$ when the temperature decreases from $x_1$ to $x_2$. It is preferred that $x_1$ and $x_2$ are the beginning and the end of the further longitudinal portion, respectively.

The preferred temperature for performing the partial oxidation depends on the comprising components of the feed stream. It is preferred that the average pressure in the tubes is in the range of 1 bar to 5 bar.

Controlling the temperature of the first product stream is preferably done by making use of a liquid cooling aid. It is more preferred that the further longitudinal portion is filled with a particulated solid material. It even more preferred that the further longitudinal portion is embedded in a further metal plate. It is further preferred that the further metal plate is in thermal contact with the liquid cooling aid. It is even further preferred that the additional longitudinal portion, the further longitudinal portion, the liquid cooling aid, the particulated solid material, and the further metal plate are located in a first reactor, as described in a previous section.

As an extension to the preferred method of cooling, it is further preferred to either choose a particulated solid material with a specific thermal conductivity, alternatively increasing the length of the further longitudinal portion, alternatively increasing the amount of particulated solid material in the further longitudinal portion, alternatively controlling the pressure in the further longitudinal portion, alternatively varying the size of the particulated solid material, or alternatively, combining any number of these possibilities.

If a particulated solid material is used as the means to achieve $R_p > R_e$, it is preferred that the particulated solid material disclosed for the first reactor is used. It is further preferred that the additional longitudinal portion and the further longitudinal portion are part of the tubes of any of the disclosed embodiments of the first reactor.

The invention is now illustrated by non-limiting examples and exemplifying embodiments, as well as figures. Note that the figures are not drawn to scale.

The optional gas cooler

In view of a safe performance of the process, the control and treatment of the gas exiting from the first outlet is particularly problematic. This reaction gas contains components, especially methacrylic acid and methacrolein, which tend to decompose or further oxidize. During decomposition, one molecule of a selected critical component produces two or more molecules of fission and oxidation products, which is accompanied by an undesirable increase in pressure. Decomposition products like methacrolein, methacrylic acid, CO, $CO_2$, acetic acid, acetone or acetaldehyde can be formed. All in all, these negative effects reduce the overall yield and efficiency of the process. Furthermore, critical conditions can occur due to this pressure increase. In various publications these effects are referred to as post combustion, blue flame and autooxidation (see Ingold, 1961, "Inhibition of Autooxidation of organic Substances, EP 30 07 69 or US 3,876,693). The state of the art describes various methods and devices to reduce these negative effects, but these proposed solutions are not applicable or insufficient for the specific problem of methacrolein oxidation to methacrylic acid according to the present invention.

Therefore, it is especially preferred that the reactor assembly comprises a gas cooler connected to the first outlet. Herein the gas stream is cooled from a temperature at the first outlet which might be in a range of 230 °C to 360 °C to a lower temperature in the range of 160 °C to 280 °C, preferably in the range of 190 °C to 270 °C, and more preferably in the range of 200 °C to 260 °C. By this preferred embodiment of the present invention any post combustion or blue flame effect can be prevented.

The gas cooler can be located inside or outside the reactor. A characteristic feature of the cooler is that it is operated with a cooling medium II which does not correspond to the cooling medium I of the catalyst bed. Preferably a Shell - Tube cooler is used, which is operated with boiler pressurized water. Here, heat respectively energy is transferred from the reaction gas to the cooling medium whereby steam might be generated.

The residence time of the gas in the reactor exit chamber and peripery to the cooler is controlled in such a way that the residence time is in a range of 0.1 sec to 10 sec, preferably of 1 sec to 7 sec.

**Figures**

List of figures

Fig. 1: schematic illustration of the cross-section of the first reactor.
Fig. 2: comprising steps of the method for producing a product.
Figs. 3.1 - 3.5: exemplary temperature profiles in the first reactor. The different profiles correspond to different experimental set-ups.

Description of figures

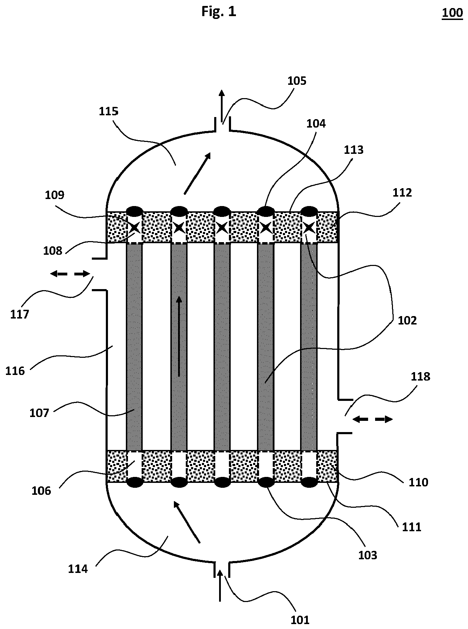

Fig. 1 shows a schematic representation of the comprising components of a first reactor 100 according to the present invention (drawing not to scale). The figure shows preferred embodiments of the present invention, and should therefore not be seen as limiting.

The solid arrows indicate the direction of flow through the first reactor 100. A feed stream flows into the first reactor 100 through a first inlet 101, and enters tubes 102 through inlet-ends 103 of the tubes 102. As the feed stream flows though the tubes 102, it is partially oxidised to a product stream, with the latter exiting the tubes 102 through the outlet-ends 104. The product stream then flows out of the first reactor 100 through a first outlet 105.

The tubes 102 have a first longitudinal portion 106. Downstream the first longitudinal portion is an additional longitudinal portion 107 that is filled with a first solid catalyst (not shown). Downstream to the additional longitudinal portion 107 is a further longitudinal portion 108 that is filled with a particulated solid material 109. Furthermore, the first longitudinal portion 106 does not contain any first solid catalyst or any particulated solid material 109.

Fig. 1 shows that the first longitudinal portion 106 and the additional longitudinal portion 107 are adjacent to each other. It is further shown that the latter longitudinal portion is adjacent to the further longitudinal portion 108. While it is preferred that the aforementioned longitudinal portions are adjacent to each other, this is not a requirement.

The first longitudinal portion 106 is embedded in a first metal plate 110. Confined by the first inlet 101 and an outer surface 111 of the first metal plate 110 is an inlet volume 114. The further longitudinal portion 108 is embedded in a further metal plate 112. Confined by the first outlet 105 and an outer surface 113 of the further metal plate 112 is an outlet volume 115. It can be seen from Fig. 1 that the first inlet 101, the inlet volume 114, the tubes 102, the outlet volume 115, and the first outlet 105 are in fluid communication with each other.

Although Fig. 1 shows the first longitudinal portion 106 is completely embedded in the first metal plate 110, the first longitudinal portion 106 can also extend beyond the first metal plate 110, either into the inlet volume 114, or into the opposite direction, or both. Similarly, the further longitudinal portion 108 can also extend beyond the further metal plate 112, either into the outlet volume 115, or into the opposite direction, or both.

A liquid cooling aid 116 circulates through the volumes between the tubes 102, and is in thermal contact with the outer surfaces (not shown) of the tubes 102. The liquid cooling aid enters and exits the first reactor 100 through openings 117 and 118. The dashed arrows indicate that either of the openings 117 and 118 can be used as the inflow and outflow-openings, thereby providing a possible means for controlling the direction of circulation of the liquid cooling aid 116.

While Fig. 1 only shows two openings 117 and 118 for the liquid cooling aid 116, the first reactor 100 can contain any number of openings. Furthermore, these openings can be arranged anywhere between the first metal plate 110 and the further metal plate 112. *E.g.,* the openings 117 and 118 should be arranged and adapted to not be in fluid communication with the inlet volume 114 and the outlet volume 115. *E.g.,* if the first reactor 100 has a largely spherocylindrical shape, the openings 117 and 118 can be located anywhere on the circumference of the first reactor 100.

Furthermore Fig. 1 shows that opening 117 is located closer to the further metal plate 112 than to the first metal plate 110, while opening 118 is located closer to the first metal plate 110 than to the further metal plate 112. If opening 117 is used as the out-flow opening and opening 118 is used as the in-flow opening, the direction of circulation of the liquid cooling aid 116 will largely be towards the left and in an upward direction. It is more preferred to have opening 118 as the in-flow opening and opening 117 as the out-flow opening. In this case of Fig. 1, the direction of circulation of the liquid cooling aid 116 will largely be towards the left and in an upward direction.

While Fig. 1 shows the feed stream / the product stream flowing in an upward direction through the first reactor 100, all possible streamlines through the first reactor fall under the scope of the present invention. This includes, *e.g.,* an embodiment wherein the first reactor 100 is arranged in such a fashion that the feed stream / the product stream will flow in a downward direction.

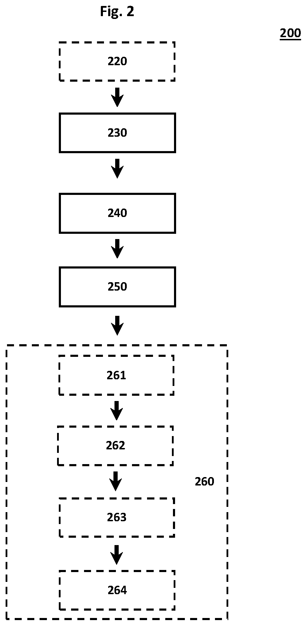

Fig. 2 shows the steps comprised in a method 200 for producing a product in the preferred form of a) preferably a polymer or b) preferably an unsaturated alkyl carboxylic compound, more preferably an unsaturated alkyl carboxylic acid or an unsaturated alkyl ester, further preferably an alk alkyl ester, even more preferably a meth alkyl ester, and in particular preferred methyl methacrylate.

In the first step 230, a gaseous feed stream that comprises a $C_3$ - $C_6$ aldehyde, preferably methacrolein, is provided. In the next step 240, the gaseous feed stream is partially oxidised on a solid catalyst to obtain a first product stream, with the solid catalyst located in an additional longitudinal portion. It is preferred that the first product stream comprises a carboxylic acid, more preferably methacrylic acid. Step 250 involves the cooling of the first product stream in a further longitudinal portion, with the temperature decreasing at a rate $R_p$. The further longitudinal portion comprises a particulated solid material.

Fig. 2 shows that the method can also contain an optional pre-step 220. In this pre-step 220, a product stream comprising a $C_3$ - $C_6$ aldehyde, preferably methacrolein, is produced from a feed stream that comprises an aldehyde pre-cursor. The product stream produced in the pre-step 220 then forms part of the feed stream 230.

In a preferred embodiment, the $C_3$ - $C_6$ aldehyde is produced in the pre-step 220 through partial oxidation. Furthermore, the resulting product stream can undergo any number of additional processes, *e.g.*, cooling and purification.

One or more subsequent processing steps 260 are also possible in the method, and may include, amongst others, in any order, and in any combination, the quenching of the first product stream 261, the purification of the first product stream 262, the esterification of the first product stream 263, and the polymerisation 264 of the ester produced in 263.

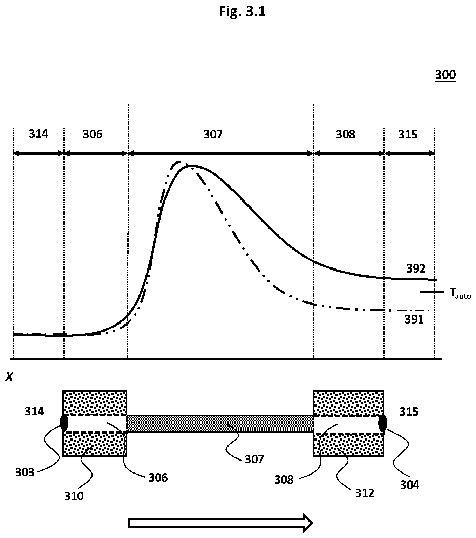

Figs. 3.1 - 3.5 show exemplary temperature profiles of a feed stream and a first product stream in a first reactor. Prior to the start of the partial oxidation, the gaseous mixture that flows into the first reactor via the first inlet is defined as the feed stream. After the start of the partial oxidation, the gaseous mixture is defined as the first product stream.

Shown below the temperature profiles is a tube that has a first longitudinal portion 306, an additional longitudinal portion 307, and a further longitudinal portion 308. The first longitudinal portion 306 is embedded in a first metal plate 310, while the further longitudinal portion 308 is embedded in a further metal plate 312. The additional longitudinal portion 307 is filled with a solid catalyst (not shown). Upstream of an inlet-end 303 of the tube is an inlet volume 314. Downstream of an outlet-end 304 of the tube is an outlet volume 315. The arrow indicates the direction of flow through the reactor.

In Figs. 3.1 - 3.5, the temperature profiles are shown for the following components of the first reactor:

314 : the inlet volume
306 : the first longitudinal portion
307 : the additional longitudinal portion
308 : the further longitudinal portion
315 : the outlet volume

Note that when it is stated that a temperature reaches a constant value, this should be understood to mean constant to a first approximation. The profiles in Figs. 3.1 - 3.5 have the same qualitative shape. In the inlet volume 314, the temperature of the feed stream remains constant. In the first longitudinal portion 306 there is a marginal increase in the temperature of the feed stream. In the additional longitudinal portion 307, partial oxidation leads to the temperature of the first product stream initially increasing sharply to a maximum value, before gradually decreasing to an end value.

After exiting the additional longitudinal portion 307, the first product stream flows through the further longitudinal portion 308 and into the outlet volume 315. The behaviour of the temperature profile in the further longitudinal portion 308 and the outlet volume 315 is determined by the presence (or absence) of particulated solid material 309. This behaviour will be discussed in more detail below. Note that in Figs. 3.1 - 3.5, $T_{auto}$ indicates the autoxidation temperature of the first product stream.

In Fig. 3.1, the first reactor does not contain any particulated solid material. Shown are two temperature profiles. The first profile 391 is for a first reactor where the solid catalyst is fresh (1-month old), and the second profile 392 is for a first reactor where the solid catalyst is used (2-years old). Both profiles show that there is a slight decrease in temperature in 308. However, in 315 the temperature remains constant. Fig. 3.1 also shows that the temperature of the product stream exiting 308 is below $T_{auto}$ when the catalyst is fresh, profile 391. However, when the catalyst has been used, profile 392, the temperature of the product stream exiting 308 is above $T_{auto}$.

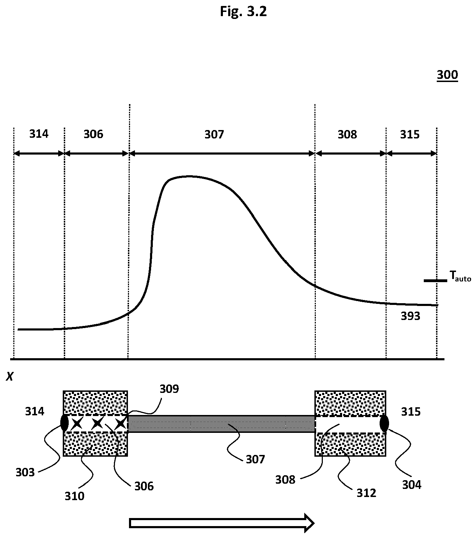

In Figs. 3.2 - 3.5, the first reactor contains the particulated solid material 309. The presence of the particulated solid material 309 leads to the temperature profiles 393 and 394. The profile 393 is for a scenario where a fresh solid catalyst is used, and the profile 394 is for a scenario where a 2-year old solid catalyst is used. The components in the first reactor that contain the particulated solid material 309 is explicitly stated in the description of Figs. 3.2 - 3.4. If not stated, then a component does not contain the particulated solid material.

Fig. 3.2 shows that the first longitudinal portion 306 is filled with the particulated solid material 309. This leads to a profile 393, with the temperature of the product stream exiting 308 being below $T_{auto}$.

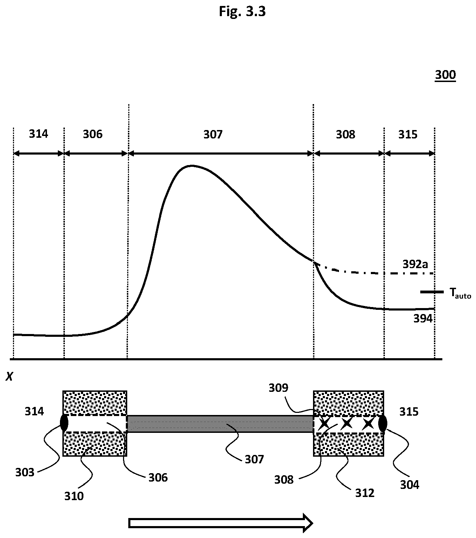

Fig. 3.3 shows that the further longitudinal portion 308 is filled with the particulated solid material 309. This results in the profile 394 where the temperature in 308 decreases rapidly, before reaching a constant value. The temperature remains constant in 315 as well. It should be noted that the temperature of the product stream exiting 308 is below $T_{auto}$. By contrast, the dashed line shows that the temperature of the product stream exiting 392a is above $T_{auto}$, where 392a corresponds to the scenario with the conditions described for the solid line 392 in Fig. 3.1 above.

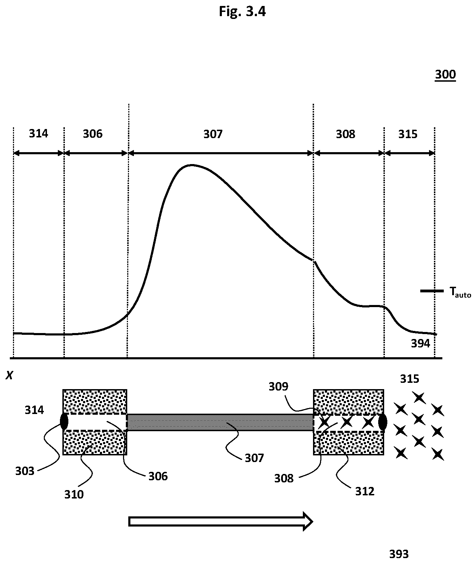

Fig. 3.4 shows that the further longitudinal portion 308 is filled with the particulated solid material 309. Fig. 3.4 further shows that the outlet volume 315 contains multiple layers of the particulated solid material 309. This results in the profile 394. More specifically, the temperature in 308 decreases rapidly, before reaching a constant value. However, in contrast to Fig. 3.3, the temperature again decreases rapidly in 315, before reaching a constant value. The temperature of the product stream exiting 308 is also below $T_{auto}$.

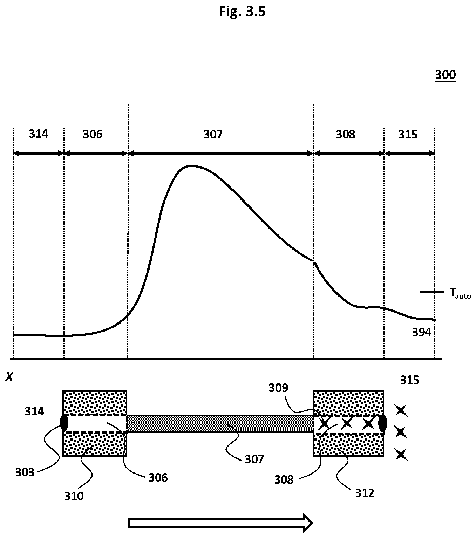

The set-up in Fig. 3.5 is similar to that of Fig. 3.4, except that the outlet volume 315 contains only a single layer of the particulated solid material 309. The main difference between profile 394 in Fig. 3.4 and profile 394 in Fig. 3.5 is that the temperature decrease in 315 is more gradual in the latter scenario. In Fig. 3.5, the temperature of the product stream exiting 308 is also below $T_{auto}$.

**Test methods**

Unless otherwise stated, all test methods that are not related to measuring the properties of a fluid are performed at a temperature of 25 °C and a pressure of 1 Atmosphere.

Length

The length of a tube or a longitudinal portion of a tube (either the first longitudinal portion, the additional longitudinal portion, or the further longitudinal portion) is measured along an imaginary line that traces the centre of the tube or the centre of the longitudinal portion of the tube.

Thickness of a layer

The thickness of a layer of particulated solid material in the inlet volume or the outlet volume can be measured using a ruler. The thickness is measured by placing one end of the ruler at the outlet-end of a tube. Furthermore, the ruler is held parallel to the tube. To obtain the average thickness, the thickness is measured at the outlet-ends of 5 % of the tubes.

Mass

The mass of the first solid catalyst or the particulated solid material can be measured using any commercially available scale.

Particle size

The size of the particles making up the particulated solid material is measured according to the standard BS 1796-1:1989. For the sieving analysis, at least 6 sieves are employed.

Specific geometric surface area

Geometric surface area determines the outer surface of an item. E.g. in case of a sphere 6 is divided by the diameter of the sphere.

Free volume

A container of known volume is filled with the particulated solid material. Water is then added to the container, until the container is filled with water. The volume of the water added to the container is divided by the volume of the container, and then multiplied by 100. This gives the free volume of the particulated solid material as a percentage. Many suitable containers are known to a person skilled in the art, and any container deemed suitable may be used. Many methods of measuring the volume of water added to the container are known to a person skilled in the art, and any method deemed suitable may be used.

Temperature

The temperature of the product stream is measured along a tube using a thermocouple placed at the centre of the tube. The thermocouple extends over the length of the tube. The thermocouple is also able to measure the temperature at 20 points in the tube. The temperature of the liquid cooling aid is also measured using a thermocouple. The thermocouple is placed at the opening though which the liquid cooling aid flows into the first reactor. Suitable thermocouples are well known to one skilled in the art, and are also commercially available.

Pressure

The pressure is measured in the inlet volume and the outlet volume. The pressure drop is therefore defined as the drop in pressure between the inlet volume and the outlet volume. The pressure is measured using a pressure load cell. Such measuring instruments are well known to one skilled in the art, and are commercially available.

Velocity (GHSV)

The mass flow rate is measured using a mass flow meter. Such measuring instruments are well known to one skilled in the art, and are commercially available. From this measurement the GHSV can be calculated. This calculation is well known to one skilled in the art.

Thermal conductivity

The thermal conductivities of suitable particulated solid material can be found in textbooks such as the Handbook of Chemistry and Physics, 56th edition (1975).

Chemical composition

The chemical composition is determined by gas chromatography. The measurement is made in the inlet volume and the outlet volume. The methods and devices for making these measurements are well known to one skilled in the art.

**Examples**

A number of experiments were performed, wherein the distribution of the particulated solid material in the first reactor was varied. Apart from the aforementioned difference, all of the experiments have the same initial set-up, as described in the following section.

Initial set-up

A feed stream is fed into a tube bundle reactor commercially obtained from Deggendorfer Werft, Germany. The feed stream is partially oxidised along additional longitudinal portions that contain a first solid catalyst to obtain a product stream comprising methacrylic acid. The choice for the first solid catalyst is described in WO 2019/141203 A1.

The feed stream flowing into the first reactor has a temperature of 235 °C, and a gas hourly space velocity of 1000 $Nm^3/m^3h$. The average pressure in the tubes is in the range of 1.25 to 2.5 bar. Furthermore, the composition of the feed stream is given in Table 1.

**Table 1:** composition of the feed stream

| Component | Mol [%] | PPM |
|---|---|---|
| Methacrolein | 3.5 | |
| Methacrylic acid | 0.1 | |
| $O_2$ | 9.1 | |
| $H_2O$ | 15.3 | |
| $N_2$ | 69.6 | |
| $CO_2$ | 2.7 | |
| Acetic acid | 0.2 | |
| Terephthalic acid | | 10 |
| MoOx | | 0.002 |

Each of the tubes in the tube bundle reactor has a first longitudinal portion, an additional longitudinal portion,

and a further longitudinal portion. The average length of the first longitudinal portions is 120 mm, the average length of the additional longitudinal portions is 3900 mm, and the average length of the further longitudinal portions is 150 mm. Furthermore, the first longitudinal portion is adjacent to the additional longitudinal portion, which in turn is adjacent to the further longitudinal portion.

For each of the tubes, the following also applies: the entire length of the additional longitudinal portion is filled with the first solid catalyst. The mass percentage of the first solid catalyst contained in the additional longitudinal portion is 99 wt-%, based on the total mass of the first solid catalyst contained in the tube. Furthermore, the mass of the first solid catalyst in a tube does not vary by more than 2 % from the average mass calculated for all the tubes. Note that the average mass is calculated by taking into account the mass of the first solid catalyst in all the tubes of all the experiments.

The first reactor also has a first metal plate and a further metal plate. The first metal plate and the further metal plate both have a thickness of 100 mm. Furthermore, the first metal plate and the further metal plate are both in thermal contact with a liquid cooling aid. The liquid cooling aid is a salt bath that is commercially available from HEF Durferrit GmbH, Germany.

The reactor has two openings for allowing the liquid cooling aid to flow into, and out of, the reactor. The in-flow opening is located closer to the first metal plate than to the further metal plate, while the out-flow opening is located closer to the further metal plate than to the first metal plate. The temperature of the liquid cooling aid flowing into the reactor is regulated to have an average value of 290 °C.

The particulated solid material used is aluminium oxide, with the size distribution of the particulated solid material being in the range of 3 mm to 7 mm. The particulated solid material is commercially available from Vereinigte Füllkärper-Fabriken GmbH & Co. KG, Germany.

Distribution of the particulated solid material

For the different experiments the distribution of the particulated solid material in the first reactor was varied. For all the experiments, the inlet volume contains no particulated solid material. Furthermore, the mass percentage of the particulated solid material in the additional longitudinal portion of a tube is less than 1 wt-%, based on the total mass of the particulated solid material in the tube.

For the different experiments, the particulated solid material is distributed through the first reactor as follows:

1. no particulated solid material in either the first longitudinal portion, the further longitudinal portion, or the outlet volume;
2. particulated solid material in the first longitudinal portion, but not in the further longitudinal portion, or the outlet volume;
3. particulated solid material in the further longitudinal portion, but not in the first longitudinal portion or the outlet volume;
4. particulated solid material in the further longitudinal portion and the outlet volume, but not in the first longitudinal portion. The thickness of the layer of particulated solid material in the outlet volume is 250 mm.
5. particulated solid material in the further longitudinal portion and the outlet volume, but not in the first longitudinal portion. The thickness of the layer of particulated solid material in the outlet volume is 50 mm.

The following also applies to all experiments: when the first longitudinal portion or the further longitudinal portion contains particulated solid material, then the entire length of the applicable longitudinal portion is filled with the particulated solid material. Furthermore, the mass of the particulated solid material in a tube does not vary by more than 2 % from the average mass calculated for all the tubes. Note that the average mass is calculated by taking into account the mass of the particulated solid material in all the tubes of all the experiments.

When particulated solid material is placed in the outlet volume, as in Experiments 4 and 5, the particulated solid material is poured randomly over the further metal plate, and then levelled so that the thickness of the layer of particulated solid material in the outlet volume is, to a first approximation, constant. Furthermore, the particulated solid material in the outlet volume is in the form of spheres with a diameter of 5 mm.

Experimental results

The results for the experiments are summarised in Table 2. Table 2 also indicates whether the particulated solid material is present in a given part of the first reactor. Note that both Experiment 4 and Experiment 5 contain particulated solid material in the outlet volume. However, the amount in Experiment 4 is more compared to the amount in Experiment 5.

Note that the results are for a first solid catalyst that has been in operation for 2 years. The exceptions are Experiments 1a and 2, which both show the results for a fresh catalyst. Furthermore, Table 2 also indicates the number of the accompanying figure exemplifying the temperature profile of the feed stream / product stream in the first reactor.

**Table 2**: the distribution of the particulated solid material in the first reactor, as well as a comparison of the experimental results.

| Example | 1a | 1b | 2 | 3 | 4 | 5 |
|---|---|---|---|---|---|---|
| Contains particulated solid material | | | | | | |
| Inlet volume | - | - | - | - | - | - |
| First longitudinal portion | - | - | Yes | - | - | - |
| Additional longitudinal portion | - | - | - | - | - | - |
| Further longitudinal portion | - | - | - | Yes | Yes | Yes |
| Outlet volume | - | - | - | - | More | Less |
| | | | | | | |
| Catalyst | Fresh | Used | Fresh | Used | Used | Used |
| | | | | | | |
| Experimental results | | | | | | |
| $T_{out} < T_{auto}$ | Yes | No | Yes | Yes | Yes | Yes |
| $T_{out}$ (°C) | 309.5 | 320.3 | 309.2 | 309.4 | 305.7 | 309.6 |
| $R$ (°C/cm) | 0.08 | 0.09 | 0.08 | 0.95 | 0.92 | 0.93 |
| Downtime | 1 | 4 | 5 | 1 | 3 | 2 |
| Safety | 1 | 3 | 3 | 1 | 3 | 1 |
| Yield difference (%) | - | -0.5 | -3.1 | + 1.5 | - 1.4 | + 0.6 |
| ΔP (mbar) | 250.9 | 254.1 | 262.8 | 261.5 | 272.3 | 264.1 |
| | | | | | | |
| Temperature profile shown in Fig. | 3.1 (dotted) | 3.1 (straight) | 3.2 | 3.3 | 3.4 | 3.5 |

Regarding the results shown in Table 2, the following should be noted:

- $T_{out} < T_{auto}$: indicates whether the temperature in the outlet volume $T_{out}$ is below the autoxidation temperature $T_{auto}$.

- $T_{out}$: the temperature of the product stream in the outlet volume.

- $R$: the rate at which the temperature of the product stream decreases in the additional longitudinal portion.

- Downtime: the downtime required when running the first reactor. The results are ranked, with 1 indicating the least downtime, and 5 indicating the most downtime.

- Safety: indicated how safely the first reactor can be run. The results are ranked, with 1 being the safest, and 5 being the least safe.

- Yield difference: the yield of Experiment la, wherein no particulated solid material is present in the reactor, is used as a baseline. Positive values indicate an increase in yield, and negative values indicate a decrease in yield, when compared to the yield of Experiment 1b.

- $\varDelta P$: the decrease in pressure between the inlet volume and the outlet volume. A smaller value is preferred.

**Reference list**

| | |
|---|---|
| 100 | First reactor |
| 101 | First inlet |
| 102 | Tubes |
| 103 | Inlet-ends of tube |
| 104 | Outlet-ends of tube |
| 105 | First outlet |
| 106 | First longitudinal portion |
| 107 | Additional longitudinal portion |
| 108 | Further longitudinal portion |
| 109 | Particulated solid material |
| 110 | First metal plate |
| 111 | Outer surface of first metal plate |
| 112 | Further metal plate |
| 113 | Outer surface of further metal plate |
| 114 | Inlet volume |
| 115 | Outlet volume |
| 116 | Liquid cooling aid |
| 117 | Opening for liquid cooling aid |
| 118 | Opening for liquid cooling aid |

| | |
|---|---|
| 200 | Method for producing a product |
| 220 | Optional production of $C_3$ - $C_6$ aldehyde |
| 230 | Provide gaseous feed stream |
| 240 | Partial oxidation of feed stream |
| 250 | Cooling of first product stream |
| 260 | Optional process steps |
| 261 | Quenching of first product stream |
| 262 | Purification of first process stream |
| 263 | Esterification of process stream |
| 264 | Polymerisation of ester produced in 262 |

| | |
|---|---|
| 303 | Inlet-end |
| 304 | Outlet-end |
| 306 | First longitudinal portion |
| 307 | Additional longitudinal portion |
| 308 | Further longitudinal portion |
| 309 | Particulated solid material |
| 310 | First metal plate |
| 312 | Further metal plate |
| 314 | Inlet volume |
| 315 | Outlet volume |
| 391 | Temperature profile without particulated solid material: fresh catalyst |
| 392 | Temperature profile without particulated solid material: used catalyst |
| 393 | Temperature profile with particulated solid material: fresh catalyst |
| 394 | Temperature profile with particulated solid material: used catalyst |

**Claims**

1. A reactor assembly arranged in fluid communication comprising a first reactor for the partial oxidation of a $C_3$ - $C_6$ aldehyde, the first reactor comprising as reactor components

a) a first inlet;
b) downstream the first inlet an inlet volume;
c) downstream the inlet volume a first metal plate;
d) downstream the first metal plate a further metal plate;
e) downstream the further metal plate an outlet volume;
f) downstream the outlet volume a first outlet;
g) a plurality of tubes with a tube length, wherein each of the tubes

(i) is located between the inlet volume and the outlet volume,
(ii) comprises an inlet-end,
(iii) comprises an outlet-end,
(iv) comprises an outer surface,
(v) comprises an inner surface,
(vi) comprises an inner volume confined by the inner surface of the tube; and wherein at least 50 % of the tubes each

[I] comprise a first longitudinal portion, wherein the first longitudinal portion is positioned in the first metal plate,
[II] comprise an additional longitudinal portion, wherein the additional longitudinal portion

(a) is arranged downstream the first longitudinal portion, and
(b) comprises a first solid catalyst,

[III] comprise a further longitudinal portion, wherein the further longitudinal portion

A) is arranged downstream the additional longitudinal portion,
B) is positioned in the further metal plate,
C) comprises an inert particulated solid material, wherein the particulated solid material

(I) is adapted and arranged to dissipate heat,
(II) is gas-permeably adapted and arranged;

h) a liquid cooling aid, wherein a portion of the outer surfaces of the tubes are arranged and adapted to be contacted with the liquid cooling aid, with the portion being at least 50 % of the length of the additional longitudinal portion;

and wherein,
an average thickness of any layer comprising the particulated solid material present in the outlet volume, the inlet volume, or both, is less than 10 % of the average length of the further longitudinal portions.

2. The reactor assembly according to claim 1, wherein at least one or all of the following applies:

a) the mass percentage of the particulated solid material present in the first longitudinal portion is less than 5 wt-%, based on the total mass of the particulated solid material contained in the tube;
b) the mass percentage of the first solid catalyst contained in the first longitudinal portion is less than 5 wt-%, based on the total mass of the first solid catalyst contained in the tube.

3. The reactor assembly according to any of the preceding claims, wherein at least 30 % of the length of the further longitudinal portions is embedded in the further metal plate.

4. The reactor assembly according to any of the preceding claims, wherein the further longitudinal portions are in thermal contact with the further metal plate.

5. The reactor assembly according to any of the preceding claims wherein the further metal plate is in thermal contact with the liquid cooling aid.

6. The reactor assembly according to any of the preceding claims, wherein at least one or all of the following applies:

a) the mass percentage of the first solid catalyst contained in the additional longitudinal portion is more than 50 wt-%, based on the total mass of the first solid catalyst contained in the tube;
b) the ratio of a mass of the solid first catalyst to a mass of the particulated solid material, both contained in the further longitudinal portion, is 1:x, where x is at least 10. Here x represents the mass of the particulated solid material.

7. The reactor assembly according to any of the preceding claims, wherein the particulated solid material comprises one or more components selected from the group consisting of metals and metal oxides.

8. The reactor assembly according to any of the preceding claims, wherein the particulated solid material has at least one or all of the following physical properties:

a) thermal conductivity in the range of 0.01 W/m/°C to 500 W/m/°C;
b) specific geometric surface area in the range of 20 $m^2/m^3$ to 2000 $m^2/m^3$;
c) free volume in the range of 24 % to 99 %; or
d) particle size distribution in the range of 0.01 mm to 100 mm.

9. The reactor assembly according to any of the preceding claims, wherein the first metal plate, the further metal plate, or both, have independently from each other a thickness in the range of 10 mm to 300 mm.

10. The reactor assembly according to any of the preceding claims, wherein the reactor assembly comprises a gas cooler connected to the first outlet.

11. A method for producing a product, wherein the method comprises the following process steps:

a) providing a gaseous feed stream, wherein the feed stream comprises an $C_3$ - $C_6$ aldehyde;
b) partially oxidising the $C_3$ - $C_6$ aldehyde in the feed stream in the presence of a solid catalyst to obtain a first product stream comprising a partial oxidation product, wherein the partial oxidation is performed along an additional longitudinal portion, and
c) cooling the first product stream along a further longitudinal portion, wherein

(i) the further longitudinal portion comprises a particulated solid material,
(ii) the temperature of the product stream in the further longitudinal portion decreases at a rate $R_p$; and

wherein
$R_p$ is larger than a rate of temperature decrease $R_e$, wherein the rate $R_e$ is for a scenario where the further longitudinal portion does not comprise the particulated solid material.

12. The method according to claim 10, wherein $R_p$ is at least 2 times larger than $R_e$.

13. The method according to any of the claims 11 to 12, wherein the rate $R_p$ is in the range of 0.1 °C/cm to 5 °C/cm.

14. The method according to any of the claims 11 to 13, wherein the temperature of the first product stream that exits the further longitudinal portion is below the autoxidation temperature of the first product stream.

15. The method according to any of the claims 11 to 14, wherein the temperature of the first product stream that exits the further longitudinal portion is in the range of > 230 °C to 360 °C.

16. The method according to claim 15, wherein the product stream that exits the first outlet is fed into a gas cooler and wherein the temperature of the product stream that exits the gas cooler is in the range of 160 °C to 280 °C.

17. The method according to any of the claims 11 to 16, wherein the product is methacrylic acid or methyl methacrylate.

Fig. 1    100

**Fig. 2**

<u>200</u>

```
┌ ─ ─ ─ ─ ─ ─ ─ ─ ┐
│       220        │
└ ─ ─ ─ ─ ─ ─ ─ ─ ┘
         │
         ▼
┌─────────────────┐
│       230        │
└─────────────────┘
         │
         ▼
┌─────────────────┐
│       240        │
└─────────────────┘
         │
         ▼
┌─────────────────┐
│       250        │
└─────────────────┘
         │
         ▼
┌ ─ ─ ─ ─ ─ ─ ─ ─ ─ ─ ─ ─ ┐
│  ┌ ─ ─ ─ ─ ─ ─ ─ ─ ┐    │
│  │      261        │    │
│  └ ─ ─ ─ ─ ─ ─ ─ ─ ┘    │
│           │             │
│           ▼             │
│  ┌ ─ ─ ─ ─ ─ ─ ─ ─ ┐    │
│  │      262        │    │
│  └ ─ ─ ─ ─ ─ ─ ─ ─ ┘    │  260
│           │             │
│           ▼             │
│  ┌ ─ ─ ─ ─ ─ ─ ─ ─ ┐    │
│  │      263        │    │
│  └ ─ ─ ─ ─ ─ ─ ─ ─ ┘    │
│           │             │
│           ▼             │
│  ┌ ─ ─ ─ ─ ─ ─ ─ ─ ┐    │
│  │      264        │    │
│  └ ─ ─ ─ ─ ─ ─ ─ ─ ┘    │
└ ─ ─ ─ ─ ─ ─ ─ ─ ─ ─ ─ ─ ┘
```

Fig. 3.1

**Fig. 3.2**

**Fig. 3.3**

300

314  306  307  308  315

392a

T_auto

394

X

309

314  315

303  310  306  307  308  312  304

Fig. 3.4

300

314 | 306 | 307 | 308 | 315

$T_{auto}$

394

X

309

315

314

303

310

306

307

308

312

393

**Fig. 3.5**

**EUROPEAN SEARCH REPORT**

Application Number

EP 20 16 9126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 532 325 A1 (NIPPON CATALYTIC CHEM IND [JP]) 17 March 1993 (1993-03-17) * page 3, line 51 - page 4, line 18; claims; figures 1,4 * * page 5, line 40 - page 6, line 15 * | 1-10 | INV. B01J8/06 |
| X | US 2013/274508 A1 (DECOURCY MICHAEL S [US] ET AL) 17 October 2013 (2013-10-17) | 11,12, 14-17 | |
| Y | * paragraph [0103]; claims 91-97; figures * | 1-10 | |
| X | EP 0 271 299 A2 (BRITISH PETROLEUM CO PLC [GB]) 15 June 1988 (1988-06-15) | 11-14 | |
| Y | * column 4, line 58 - column 5, line 11; claim 1; figure 1 * | 1-10, 15-17 | |
| X | US 2019/270688 A1 (BOS ALOUISIUS NICOLAAS RENÉE [NL] ET AL) 5 September 2019 (2019-09-05) | 1-4, 11-13, 15,16 | |
| Y | * paragraph [0037] * * paragraphs [0021] - [0023], [0037] - [0038], [0043], [0047], [0048]; figures * | 14 | |
| | | | TECHNICAL FIELDS SEARCHED (IPC) |
| | | | B01J |
| X | EP 0 990 636 A1 (ROHM & HAAS [US]) 5 April 2000 (2000-04-05) * paragraphs [0047] - [0051], [0054] - [0056]; claims; figures 2-3 * | 1,2,11, 14-16 | |
| X | EP 0 911 313 A1 (NIPPON CATALYTIC CHEM IND [JP]) 28 April 1999 (1999-04-28) * paragraphs [0015], [0016], [0022] - [0028], [0040]; claim 1 * | 1,11,14 | |
| X | WO 2006/098601 A1 (LG CHEMICAL LTD [KR]) 21 September 2006 (2006-09-21) | 11-17 | |
| Y | * paragraphs [0014] - [0033] * | 1-10 | |

-/--

The present search report has been drawn up for all claims

| Place of search | Date of completion of the search | Examiner |
|---|---|---|
| Munich | 10 September 2020 | Kurtulan Dogan, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 16 9126

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/021238 A1 (YAMAGISHI MASAHIKO [JP] ET AL) 24 January 2008 (2008-01-24) * paragraphs [0061] - [0066]; claims; figures * | 11-17 | |
| Y | US 2011/166304 A1 (ZANTHOFF HORST-WERNER [DE] ET AL) 7 July 2011 (2011-07-07) * paragraphs [0010] - [0014], [0065], [0066], [0070], [0079], [0099]; figures * | 1-17 | |
| Y | EP 1 697 278 A1 (LG CHEMICAL LTD [KR]) 6 September 2006 (2006-09-06) * paragraphs [0052], [0053], [0054], [0055], [0056] - [0059], [0066] - [0069]; figure 1 * | 1-17 | |
| Y | EP 1 412 077 A1 (BP CORP NORTH AMERICA INC [US]) 28 April 2004 (2004-04-28) * the whole document * | 1-17 | |
| A,D | DE 100 47 693 A1 (BASF AG [DE]) 11 April 2002 (2002-04-11) * the whole document * | 1-17 | TECHNICAL FIELDS SEARCHED (IPC) |

The present search report has been drawn up for all claims

| Place of search | Date of completion of the search | Examiner |
|---|---|---|
| Munich | 10 September 2020 | Kurtulan Dogan, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EP 3 892 367 A1**

## ANNEX TO THE EUROPEAN SEARCH REPORT
## ON EUROPEAN PATENT APPLICATION NO.

EP 20 16 9126

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2020

| Patent document cited in search report | | Publication date | Patent family member(s) | | Publication date |
|---|---|---|---|---|---|
| EP 0532325 | A1 | 17-03-1993 | CA | 2078060 A1 | 13-03-1993 |
| | | | DE | 69206542 T2 | 15-05-1996 |
| | | | EP | 0532325 A1 | 17-03-1993 |
| | | | JP | 2778878 B2 | 23-07-1998 |
| | | | JP | H05194452 A | 03-08-1993 |
| | | | US | 5292904 A | 08-03-1994 |
| US 2013274508 | A1 | 17-10-2013 | BR | 112015006503 A2 | 04-07-2017 |
| | | | CN | 104661738 A | 27-05-2015 |
| | | | CN | 106955646 A | 18-07-2017 |
| | | | EP | 2922624 A2 | 30-09-2015 |
| | | | EP | 3682967 A1 | 22-07-2020 |
| | | | JP | 6290219 B2 | 07-03-2018 |
| | | | JP | 6728253 B2 | 22-07-2020 |
| | | | JP | 2015535826 A | 17-12-2015 |
| | | | JP | 2018111696 A | 19-07-2018 |
| | | | KR | 20150060895 A | 03-06-2015 |
| | | | KR | 20200070429 A | 17-06-2020 |
| | | | KR | 20200070433 A | 17-06-2020 |
| | | | MX | 341436 B | 18-08-2016 |
| | | | SG | 102016108920 A | 27-02-2017 |
| | | | SG | 10201911923S A | 27-02-2020 |
| | | | SG | 11201502015W A | 29-04-2015 |
| | | | TW | 201412400 A | 01-04-2014 |
| | | | TW | 201722550 A | 01-07-2017 |
| | | | US | 2013274508 A1 | 17-10-2013 |
| | | | US | 2016303532 A1 | 20-10-2016 |
| | | | US | 2018078919 A1 | 22-03-2018 |
| | | | US | 2019201862 A1 | 04-07-2019 |
| | | | WO | 2014046829 A2 | 27-03-2014 |
| | | | ZA | 201501789 B | 28-09-2016 |
| EP 0271299 | A2 | 15-06-1988 | AU | 598074 B2 | 14-06-1990 |
| | | | CA | 1329704 C | 24-05-1994 |
| | | | EP | 0271299 A2 | 15-06-1988 |
| | | | JP | S63218792 A | 12-09-1988 |
| | | | NO | 171045 B | 12-10-1992 |
| | | | NZ | 222835 A | 28-08-1990 |
| | | | ZA | 879257 B | 30-08-1989 |
| US 2019270688 | A1 | 05-09-2019 | AU | 2017304583 A1 | 07-03-2019 |
| | | | BR | 112019001106 A2 | 30-04-2019 |
| | | | CA | 3031565 A1 | 01-02-2018 |
| | | | CN | 109476564 A | 15-03-2019 |
| | | | EA | 201990379 A1 | 28-06-2019 |
| | | | EP | 3490962 A1 | 05-06-2019 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 20 16 9126

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2020

| Patent document cited in search report | | Publication date | Patent family member(s) | | Publication date |
|---|---|---|---|---|---|
| | | | KR 20190038578 A | | 08-04-2019 |
| | | | US 2019270688 A1 | | 05-09-2019 |
| | | | WO 2018019761 A1 | | 01-02-2018 |
| EP 0990636 | A1 | 05-04-2000 | BR 9900847 A | | 09-05-2000 |
| | | | CN 1249300 A | | 05-04-2000 |
| | | | DE 69910930 T2 | | 22-07-2004 |
| | | | EP 0990636 A1 | | 05-04-2000 |
| | | | JP 2000103761 A | | 11-04-2000 |
| | | | KR 20000022591 A | | 25-04-2000 |
| | | | MX PA99001856 A | | 15-07-2005 |
| | | | TW I239328 B | | 11-09-2005 |
| | | | US 6384274 B1 | | 07-05-2002 |
| EP 0911313 | A1 | 28-04-1999 | BR 9804243 A | | 11-04-2000 |
| | | | CN 1215718 A | | 05-05-1999 |
| | | | DE 69801797 T2 | | 23-05-2002 |
| | | | EP 0911313 A1 | | 28-04-1999 |
| | | | ID 21140 A | | 29-04-1999 |
| | | | JP 3948798 B2 | | 25-07-2007 |
| | | | JP H11130722 A | | 18-05-1999 |
| | | | KR 19990037343 A | | 25-05-1999 |
| | | | MX 207634 B | | 29-04-2002 |
| | | | MY 120056 A | | 30-08-2005 |
| | | | SG 70657 A1 | | 22-02-2000 |
| | | | TW 460455 B | | 21-10-2001 |
| | | | US 6069271 A | | 30-05-2000 |
| WO 2006098601 | A1 | 21-09-2006 | CN 101142163 A | | 12-03-2008 |
| | | | EP 1858833 A1 | | 28-11-2007 |
| | | | JP 4659881 B2 | | 30-03-2011 |
| | | | JP 2008535802 A | | 04-09-2008 |
| | | | KR 20060101323 A | | 22-09-2006 |
| | | | TW 200700374 A | | 01-01-2007 |
| | | | US 2006211884 A1 | | 21-09-2006 |
| | | | US 2011008218 A1 | | 13-01-2011 |
| | | | WO 2006098601 A1 | | 21-09-2006 |
| US 2008021238 | A1 | 24-01-2008 | BR PI0418881 A | | 27-11-2007 |
| | | | CN 1697811 A | | 16-11-2005 |
| | | | JP 2005336142 A | | 08-12-2005 |
| | | | RU 2355673 C2 | | 20-05-2009 |
| | | | US 2008021238 A1 | | 24-01-2008 |
| | | | WO 2005115961 A1 | | 08-12-2005 |
| US 2011166304 | A1 | 07-07-2011 | BR PI0914143 A2 | | 20-10-2015 |

EPO FORM P0459

For more details about this annex : see Official Journal of the European Patent Office, No. 12/82

page 2 of 3

## ANNEX TO THE EUROPEAN SEARCH REPORT
## ON EUROPEAN PATENT APPLICATION NO.

EP 20 16 9126

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2020

| Patent document cited in search report | | | Publication date | Patent family member(s) | | Publication date |
|---|---|---|---|---|---|---|
| | | | | CN | 102137833 A | 27-07-2011 |
| | | | | DE | 102008044946 A1 | 04-03-2010 |
| | | | | EP | 2315738 A2 | 04-05-2011 |
| | | | | JP | 2012500820 A | 12-01-2012 |
| | | | | US | 2011166304 A1 | 07-07-2011 |
| | | | | WO | 2010023053 A2 | 04-03-2010 |
| | | | | ZA | 201102225 B | 30-11-2011 |
| EP 1697278 | A1 | 06-09-2006 | | CN | 1871189 A | 29-11-2006 |
| | | | | CN | 101429109 A | 13-05-2009 |
| | | | | EP | 1697278 A1 | 06-09-2006 |
| | | | | IN | 235864 B | 04-09-2009 |
| | | | | JP | 4477640 B2 | 09-06-2010 |
| | | | | JP | 2007523054 A | 16-08-2007 |
| | | | | KR | 20050065425 A | 29-06-2005 |
| | | | | TW | I288127 B | 11-10-2007 |
| | | | | US | 2005143601 A1 | 30-06-2005 |
| | | | | WO | 2005061414 A1 | 07-07-2005 |
| EP 1412077 | A1 | 28-04-2004 | | AP | 1819 A | 10-01-2008 |
| | | | | AT | 529183 T | 15-11-2011 |
| | | | | AU | 2002322502 B2 | 22-05-2008 |
| | | | | BR | 0211454 A | 17-08-2004 |
| | | | | CA | 2451090 A1 | 13-02-2003 |
| | | | | CN | 1531458 A | 22-09-2004 |
| | | | | EA | 200400768 A1 | 30-12-2004 |
| | | | | EP | 1412077 A1 | 28-04-2004 |
| | | | | EP | 2314372 A1 | 27-04-2011 |
| | | | | ES | 2373310 T3 | 02-02-2012 |
| | | | | JP | 4323950 B2 | 02-09-2009 |
| | | | | JP | 2005518265 A | 23-06-2005 |
| | | | | KR | 20040019101 A | 04-03-2004 |
| | | | | KR | 20100019540 A | 18-02-2010 |
| | | | | KR | 20110017463 A | 21-02-2011 |
| | | | | MX | PA04000890 A | 21-05-2004 |
| | | | | MY | 142104 A | 15-09-2010 |
| | | | | TW | 579421 B | 11-03-2004 |
| | | | | US | 2003068261 A1 | 10-04-2003 |
| | | | | US | 2009043114 A1 | 12-02-2009 |
| | | | | US | 2011144356 A1 | 16-06-2011 |
| | | | | WO | 03011449 A1 | 13-02-2003 |
| DE 10047693 | A1 | 11-04-2002 | | NONE | | |

EPO FORM P0459

For more details about this annex : see Official Journal of the European Patent Office, No. 12/82

**EP 3 892 367 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 3042468 A1
- DE 10047693 A1
- DE 19806810 A1
- WO 2008145418 A1
- WO 2004073857 A1
- WO 2019141203 A1
- US 5583084 A
- EP 1995232 B1
- US 5292797 A
- US 4562234 A
- US 5773505 A
- US 5612417 A
- US 4952455 A
- US 4948668 A
- US 4239671 A
- EP 300769 A
- US 3876693 A

**Non-patent literature cited in the description**

- **KRILL ; OFFERMANNS ; RÜHLING.** *Chem. Unserer Zeit,* 2019, 53
- **INGOLD.** *Inhibition of Autooxidation of organic Substances,* 1961
- Handbook of Chemistry and Physics. 1975